# EUROPEAN PATENT SPECIFICATION

(11) **EP 3 972 554 B1**
(45) Date of publication and mention of the grant of the patent: **21.10.2026**
(21) Application number: 20719959.7
(22) Date of filing: 07.04.2020
(51) Int. Cl.: A61K 8/11, C11D 17/00, C11D 3/37, C11D 3/50, C11D 3/22, A61Q 5/12, A61Q 13/00, A61K 8/898, A61K 8/73, A61K 8/40, A61K 8/362, B01J 13/22

(54) **CORE-SHELL ENCAPSULATED COMPOSITION COMPRISING A BENEFIT AGENT**
KERN-SCHALE-VERKAPSELTE ZUSAMMENSETZUNG MIT EINEM PFLEGEMITTEL
COMPOSITION ENCAPSULÉE À COEUR-ENVELOPPE COMPRENANT UN AGENT BÉNÉFIQUE

(30) Priority: 20.05.2019 GB 201907053
(43) Date of publication of application: 30.03.2022
(73) Proprietor: Givaudan SA, 1214 Vernier (CH)
(72) Inventor: DEMBELE-KUNTZMANN, Fatimata, 92600 Asnieres-sur-Seine (FR); DENIGOT, Marion, 78600 Maisons Laffitte (FR); HARRISON, Ian Michael, 78300 Poissy (FR)
(74) Representative: Global Patents
(86) International application number: PCT/EP2020/059827
(87) International publication number: WO 2020/233887

(56) References cited:
- WO-A1-2016/193435
- WO-A1-2018/019896
- WO-A1-2019/121736
- WO-A2-2013/083760
- JP-A- 2013 027 322
- US-A1- 2011 118 161
- US-A1- 2016 325 259
- US-B2- 9 963 661

## Description

The present invention is concerned with encapsulated compositions comprising at least one core-shell microcapsule. The invention also relates to a method for preparing such encapsulated compositions and to their use to enhance the performance of a benefit agent in a consumer product. Furthermore, the present invention refers to a polymeric stabilizer, as well as to a use of such a polymeric stabilizer in the encapsulation of a benefit agent.

It is known to incorporate encapsulated benefit agents in consumer products, such as household care, personal care and fabric care products. Benefit agents include for example fragrances, cosmetic agents, food ingredients, nutraceuticals, drugs and substrate enhancers.

Microcapsules that are particularly suitable for delivery of such benefit agents are core-shell microcapsules, wherein the core usually comprises the benefit agent and the shell is impervious or partially impervious to the benefit agent. Generally, these microcapsules are employed in aqueous media and the encapsulated benefit agents are hydrophobic. A broad selection of shell materials can be used, provided the shell material is impervious or partially impervious to the encapsulated benefit agent.

Benefit agents are encapsulated for a variety of reasons. Microcapsules can isolate and protect such materials from external suspending media, such as consumer product bases, in which they may be incompatible or unstable. They are also used to assist in the deposition of benefit agents onto substrates, such as skin or hair, or also fabrics or hard household surfaces in case of perfume ingredients. They can also act as a means of controlling the spatio-temporal release of a benefit agent.

A wide variety of encapsulating media as well as benefit agents suitable for the preparation of encapsulated compositions has been proposed in the prior art. Such encapsulating media include synthetic resins made from polyamides, polyureas, polyurethanes, polyacrylates, melamine-derived resins, or mixtures thereof.

As for suitable benefit agents, it is generally accepted that certain physicochemical characteristics of an agent, most notably its clogP, will influence whether and to what extent it can be encapsulated, and once encapsulated, its propensity to remain in the core without substantial leakage during manufacture and storage.

In the hands of the skilled formulator, the judicious selection of both the shell-forming and core materials can result in microencapsulated compositions that are stable in many consumer products, and which allow modulation of benefit agent release over time. However, even the use of well-established shell chemistries in combination with an appropriate formulation of core material, the formulator is faced with a difficult trade-off between ensuring on one hand that the microcapsules are sufficiently robust as to be stable and not leaky during manufacture and storage, and on the other that there is acceptable release of the core contents as desired in application. Another problematic aspect of encapsulating benefit agents is the control of undesired side reactions of shell-forming compounds with the materials to be encapsulated during capsule formation.

By way of example, WO 2016/207187 A1 discloses aminoplast core-shell microcapsules. These microcapsules have excellent properties, both in manufacture and application.

WO2013083760A2 discloses core-shell microcapsules with shells derived from at least two alkoxysilanes. JP2013027322A discloses core-shell microcapsules with shells derived from a silane compound having an amino group.

However, nowadays consumers are increasingly concerned about using materials obtained from non-renewable sources, such as synthetic petrochemicals. In other words, consumers tend to favor materials the origin of which is more sustainable in terms of environment and resource protection. Nevertheless, it is generally difficult to use natural materials or materials derived from nature to address all aspects of benefit agent encapsulation. In particular, the means of forming capsules that can encapsulate with high encapsulation efficiency and that are sufficiently impervious to benefit agents during storage has proved to be elusive.

It is therefore a problem underlying the present invention to overcome the above-mentioned shortcomings in the prior art. In particular, it is a problem underlying the present invention to provide encapsulated compositions of the above-mentioned kind that are more sustainable, in particular by comprising increased levels of natural materials or materials derived from nature, whilst keeping the desired benefit-agent release properties, both during manufacture, storage and in application. Furthermore, the compositions should be producible in an operationally safe, robust and cost-efficient process.

These problems are solved by an encapsulated composition according to the present invention. Such a composition comprises at least one core-shell microcapsule. The at least one core-shell microcapsule comprises a core comprising at least one benefit agent and a shell surrounding the core. The shell comprises a polymeric stabilizer that is formed by combination of a polymeric surfactant with at least one aminosilane, according to claim 1. The polymeric surfactant comprises, in particular consists of, a polysaccharide comprising carboxylic acid groups.

In the present context, the term *"benefit agent"* refers to any substance which, when added to a product, may improve the perception of this product by a consumer or may enhance the action of this product in an application. Typical benefit agents include perfume ingredients, flavor ingredients, cosmetic ingredients, bioactive agents (such as bactericides, insect repellents and pheromones), substrate enhancers (such as silicones and brighteners), enzymes (such as lipases and proteases), dyes, pigments and nutraceuticals.

The term *"polymeric surfactant"* refers to a polysaccharide or a mixture comprising at least one polysaccharide that has the property of lowering the interfacial tension between an oil phase and an aqueous phase, when dissolved in one or both of the phases. This ability to lower interfacial tension is called *"interfacial activity".*

The term *"formed by combination"* in the present context means that the polymeric surfactant and the at least one aminosilane are brought in contact with each other to generate the polymeric stabilizer. Without being bound to any theory, this formation can be the result of an interaction between the polymeric surfactant and the at least one aminosilane, such as through dispersion forces, electrostatic forces or hydrogen bonds. But also a chemical reaction, in strict sense, to form covalent bonds is encompassed by this term.

In other words, the polymeric stabilizer can be regarded as an assembly, which comprises moieties derived from a polymeric surfactant and moieties derived from at least one aminosilane.

In the context of the present invention, the polymeric surfactant is soluble or dispersible in an aqueous phase or in water, respectively. This means that the individual polymeric surfactant macromolecules are substantially separated from each other in these liquids. The resulting system appears transparent or hazy when inspected by the human eye.

In addressing the problems of the prior art, it has been found that combining a polymeric surfactant as defined herein above with at least one aminosilane according to claim 1 results in the formation of a polymeric stabilizer, which is more sustainable than stabilizers known in the prior art, particular in terms of environment and resources protection. Without being bound by any theory, it is surmised that the carboxylic acid groups may interact with the at least one aminosilane in a manner mentioned hereinabove.

The polysaccharide comprising carboxylic acid groups may comprise uronic acid units, in particular hexuronic acid units. Polysaccharides having uronic acid units, in particular hexuronic acid units, are broadly available in nature.

The hexuronic acid units can be selected from the group consisting of galacturonic acid units, glucuronic acid units, in particular 4-O-methyl-glucuronic acid units, guluronic acid units and mannuronic acid units.

The polysaccharide comprising carboxylic acid groups may be branched. Branched polysaccharides comprising carboxylic acid groups have the advantage of forming more compact networks than linear polysaccharides and therefore may favor the imperviousness of the encapsulating shell, resulting in reduced leakage and greater encapsulation efficiency.

The carboxylic acid groups can be partially present in the form of the corresponding methyl ester. The percentage of carboxylic acid groups that are present in the form of the corresponding methyl ester can be from 3 % to 95 %, preferably from 4 % to 75 %, more preferably from 5 to 50 %. Alternatively, the percentage of carboxylic acid groups that are present in the form of the corresponding methyl ester can be less than 50 %.

In the context of the present invention, polysaccharides comprising carboxylic acid groups, of which 50 % or more are present in the form of the corresponding methyl ester, are referred to as *"high methoxylated".* Polysaccharides comprising carboxylic acid groups, of which less than 50 % are present in the form of the corresponding methyl ester, are referred to as *"low methoxylated".*

The carboxylic acid groups can at least partially be present in the form of the corresponding carboxylate salt, in particular the corresponding sodium, potassium, magnesium or calcium carboxylate salt.

In an alternative embodiment of the present invention, the carboxylic acid groups can at least partially be present in the form of a complex with a species selected from the group consisting of a zirconium species, a titanium species and a boron species, wherein the species are especially oxides.

Without being bound by any theory, it is surmised that presence of carboxylate salts or complexes in the polysaccharides limits their solubility in water and thereby promotes the formation of capsule shells. Furthermore, polyvalent metal species may promote intermolecular cross-linking, which may also improve the encapsulating properties of the shell.

The polysaccharides comprising carboxylic acid groups may be at least partially acylated. As with the methyl ester groups mentioned hereinabove, partial acylation of the polysaccharide units can enhance the interfacial activity of the polymeric surfactant.

The polymeric surfactant can be selected from pectin, gum arabic and an alginate. As illustrated in the examples, these polysaccharides offer a most suitable combination of solubility, viscosity and interfacial activity that make the microcapsules according to the invention particularly performing in terms of handling, storage stability and olfactive performance. The polymeric surfactant may also be hyaluronic acid.

The polymeric surfactant may cause a surface tension of less than 45 mN/m, more particularly less than 35 mN/m, still more particularly less than 25 mN/m, in a 1 wt.-% aqueous solution containing 0.01 wt.-% of sodium chloride, when measured after 1 h of equilibration at pH 4.5 at a temperature of 25 °C.

A convenient way to assess the interfacial activity of a polymeric surfactant is to measure the tension of the interface between the aqueous phase comprising the polymeric surfactant and air. This tension is called *"surface tension"* and is generally expressed in mN/m. The surface tension may be measured by a number of methods which are well known to the person skilled in the art. In context of the present invention, the surface tension is measured by the so-called Pending Drop Method.

For a given polymeric surfactant, the surface tension depends on the temperature and on the concentration of this polymeric surfactant in the aqueous phase. Furthermore, if the polymeric surfactant is a polyelectrolyte comprising cationic groups or anionic groups or is a polymer comprising groups that can form cations or anions, the surface tension additionally depends on the ionic strength and/or on the pH of the aqueous phase. The surface tension of pure water is about 72 mN/m at 25 °C.

Disclosed herein are aminosilanes selected from a compound of Formula (I).

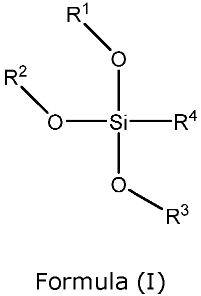

In the above Formula (I), R¹, R² and R³ are each independently C₁-C₄ linear or branched alkyl or alkenyl residues, in particular methyl or ethyl, and R⁴ is a C₁-C₁₂, preferably a C₁-C₄, linear or branched alkyl or alkenyl residue comprising an amine functional group, in particular a primary, secondary or tertiary amine.

When the functional group is a primary amine, it can be a terminal primary amine. R⁴ is then preferably a C₁-C₈, even more preferably a C₁-C₄, linear terminal primary aminoalkyl residue. Specific aminosilanes of this category are selected from the group consisting of aminomethyltriethoxysilane, 2-aminoethyltriethoxysilane, 3-aminopropyltriethoxysilane, 4-aminobutyltri-ethoxysilane, 5-aminopentyltriethoxysilane, 6-aminohexyltriethoxysilane, 7-aminohptyltriethoxysilane and 8-aminooctyltriethoxysilane.

Without being bound by any theory, it is surmised that the silane groups polycondensate with one another to form a silica network at a liquid-liquid interface that additionally stabilizes this interface.

The aminosilane in the present invention is a bipodal aminosilane. By *"bipodal aminosilane"* is meant a molecule comprising at least one amino group and two residues, each of these residues bearing at least one alkoxysilane moiety.

In particular embodiments of the present invention, the at least one bipodal aminosilane has the Formula (II).

(O-R⁴)_{(3-f)}(R³)_{f}Si-R²-X-R²-Si(O-R⁴)_{(3-f)}(R³)_{f} Formula (II)

In the above Formula (II), X stands for -NR¹-, -NR¹-CH₂-NR¹-, -NR¹-CH₂-CH₂-NR¹-, -NR¹-CO-NR¹-, or

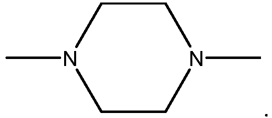

In the above Formula (II), R¹ each independently stand for H, CH₃ or C₂H₅. R² each independently stand for a linear or branched alkylene group with 1 to 6 carbon atoms. R³ each independently stand for a linear or branched alkyl group with 1 to 4 carbon atoms. R⁴ each independently stand for H or for a linear or branched alkyl group with 1 to 4 carbon atoms. f stands for 0, 1 or 2.

Bipodal aminosilanes are particularly advantageous for forming stable oil-water interfaces, compared to conventional silanes.

Examples of bipodal aminosilanes include, but are not limited to, bis(3-(triethoxysilyl)propyl)amine, N,N'-bis(3-(trimethoxysilyl)propyl)urea, bis(3-(methyldiethoxysilyl) propyl)amine, N,N'-bis(3-(trimethoxysilyl)propyl)ethane-1,2-diamine, bis(3-(methyldimethoxysilyl)propyl)-N-methylamine and N,N'-bis(3-(triethoxysilyl) propyl)piperazine.

The bipodal aminosilane can be a secondary aminosilane. Using a secondary bipodal aminosilane instead of primary aminosilane decreases the reactivity of the polymeric stabilizer with respect to electrophilic species, in particular aldehydes. Hence, benefit agents containing high levels of aldehydes may be encapsulated with a lower propensity for adverse interactions between core-forming and shell-forming materials.

The secondary bipodal aminosilane can be bis(3-(triethoxysilyl)propyl)amine. This particular secondary aminosilane has the advantage of releasing ethanol instead of more toxic and less desirable methanol during the polycondensation of the ethoxysilane groups.

Other aminosilanes may also be used in combination with the aforementioned bipodal aminosilanes, in particular the aminosilanes described hereinabove.

The aminosilane to polymeric surfactant weight ratio can be from 0.1 to 1.1, in particular from 0.2 to 0.9, even more particularly from 0.3 to 0.7, for example 0.5.

The polymeric stabilizer can be formed by combination of a polymeric surfactant with at least one aminosilane and further a polyfunctional isocyanate. Polyfunctional isocyanates may densify the arrangement of the polymeric surfactant at the oil/water interface. Without being bound by any theory, it is supposed that the polyfunctional isocyanate cross-links both aminosilanes and polysaccharides by forming polyurea and polyurethane bonds.

The polyfunctional isocyanate may be selected from alkyl, alicyclic, aromatic and alkylaromatic, as well as anionically modified polyfunctional isocyanates, with two or more (e.g. 3, 4, 5, etc.) isocyanate groups in a molecule.

Preferably, at least one polyfunctional isocyanate is an aromatic or an alkylaromatic polyfunctional isocyanate, the alkylaromatic polyfunctional isocyanate having preferably methylisocyanate groups attached to an aromatic ring. Both aromatic and methylisocyanate-substituted aromatic polyfunctional isocyanates have a superior reactivity compared to alkyl and alicyclic polyfunctional isocyanates. Among these, 2-ethylpropane-1,2,3-triyl tris((3-(isocyanatomethyl)phenyl)carbamate) is particularly preferred, because of its tripodal nature that favors the formation of intermolecular cross-links and because of its intermediate reactivity that favors network homogeneity. This alkylaromatic polyfunctional isocyanate is commercially available under the trademark Takenate D-100 N, sold by Mitsui or under the trademark Desmodur^{®} Quix175, sold by Covestro.

As an alternative to the aromatic or alkylaromatic polyfunctional isocyanate, it may also be advantageous to add an anionically modified polyfunctional isocyanate, because of the ability of such polyfunctional isocyanates to react at the oil/water interface and even in the water phase close to the oil/water interface. A particularly suitable anionically modified polyfunctional isocyanate has Formula (III).

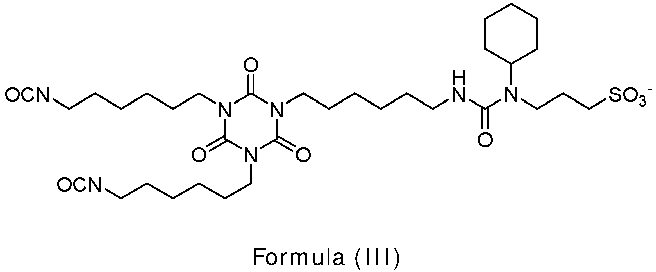

Formula (III) shows a commercially available anionically modified polyisocyanate, which is a modified isocyanurate of hexamethylene diisocyanate, sold by Covestro under the trademark Bayhydur^{®} XP2547.

In a particularly preferred embodiment of the present invention, the polymeric stabilizer is formed by combination of pectin with bis(3-(triethoxysilyl)propyl)amine. Preferably, the polymeric stabilizer is formed by combination of pectin with bis(3-(triethoxysilyl)propyl)amine and 2-ethylpropane-1,2,3-triyl tris((3-(isocyanatomethyl)phenyl)carbamate). These combinations of natural polymeric surfactant and bipodal secondary aminosilane provide particularly advantageous interface stability and release properties. The stabilized interface is sufficiently impervious to effectively encapsulate the at least one benefit agent comprised in the core. The polymeric stabilizer effectively forms a shell encapsulating the at least one perfume ingredient comprised in the core.

Core-shell microcapsules according to the present invention generally have a volume average size (d50) of 1 to 100 µm, preferably 5 to 50 µm, even more preferably 10 to 30 µm.

In another aspect, the present invention relates to an encapsulated composition, in particular a composition as described herein above. The encapsulated composition comprises at least one core-shell microcapsule. The at least one core-shell microcapsule comprises a core comprising at least one benefit agent and a shell surrounding the core. The shell comprises a polymeric stabilizer that is formed by combination of a polymeric surfactant with at least one bipodal aminosilane. The shell additionally comprises a polysaccharide, preferably a polysaccharide comprising beta (1 → 4) linked monosaccharide units, even more preferably a cellulose derivative, in particular selected form the group consisting of hydroxyethyl cellulose, hydroxpropylmethyl cellulose, cellulose acetate and carboxymethyl cellulose, preferably hydroxyethyl cellulose.

In order to avoid any doubt, the polymeric stabilizer referred to in the foregoing paragraph does not need to be a polysaccharide comprising carboxylic acid groups. In case the polymeric stabilizer referred to in the foregoing paragraph is a polysaccharide comprising carboxylic acid groups, polysaccharide additionally comprised in the shell is a further polysaccharide.

It has been found that the polymeric stabilizer is a relevant factor to the balance between microcapsule stability with respect to both perfume leakage during storage and perfume release under in-use conditions. In particular, the importance of providing additional stabilization of the oil-water interface has been recognized. The polymeric stabilizer thus provides a stable platform, which allows for the addition of additional shell materials and /or shell precursors to form novel encapsulated perfume compositions. More specifically, the addition of a polysaccharide, preferably a polysaccharide comprising beta (1 → 4) linked monosaccharide units, even more preferably a cellulose derivative, leads to highly sustainable microcapsules with an excellent release profile.

The polysaccharide may be deposited on the outer surface of the capsule shell formed by the polymeric stabilizer. This results in a multilayer shell having at least one layer of polymeric stabilizer and one layer of polysaccharide. It may improve the imperviousness of the encapsulating shell by increasing the amount of encapsulating material.

To avoid any ambiguity, the present invention is by no means restricted to a shell having sharply defined discrete layers, although this is one possible embodiment. More specifically, the layers can also be gradual and undiscrete. On the other hand, and at the other extreme, the shell can even be essentially homogenous.

The polysaccharide may react with unreacted isocyanate groups and increase the density of the cross-linked shell. But the polysaccharide may also interact with the polymeric stabilizer by physical forces, physical interactions, such as hydrogen bonding, ionic interactions, hydrophobic interactions or electron transfer interactions.

The shell additionally comprising a polysaccharide can be further stabilized with a stabilizing agent. Preferably the stabilizing agent comprises at least two carboxylic acid groups. Even more preferably, the stabilizing agent is selected from the group consisting of citric acid, benzene-1,3,5-tricarboxylic acid, 2,5-furandicarboxylic acid, itaconic acid, poly(itaconic acid) and combinations thereof.

Yet another aspect of the present invention relates to a method for preparing an encapsulated composition as described herein above. This method comprises the steps of:
a) Providing a polymeric surfactant;
b) Providing an aqueous phase;
c) Dissolving or dispersing the polymeric surfactant in the aqueous phase;
d) Providing at least one aminosilane;
e) Providing an oil phase comprising at least one benefit agent;
f) Optionally: Dissolving the at least one aminosilane in the oil phase;
g) Emulsifying the oil phase and the aqueous phase in presence of both of the polymeric surfactant and the aminosilane to form an emulsion of oil droplets in the aqueous phase;
h) Causing the at least one aminosilane and the polymeric surfactant to form a shell at the oil-water interface of the emulsified oil droplets, thereby forming a slurry of microcapsules;
i) Optionally: Adding a polysaccharide, preferably a polysaccharide comprising beta (1 → 4) linked monosaccharide units, even more preferably a cellulose derivative, in particular selected form the group consisting of hydroxyethyl cellulose, hydroxpropylmethyl cellulose, cellulose acetate and carboxymethyl cellulose, preferably hydroxyethyl cellulose, to the microcapsule slurry formed in step h),
wherein the aminosilane is a bipodal aminosilane.

Oil-in-water emulsions have the advantage of providing a plurality of droplets that may be used as template for shell formation, wherein the shell is built around each of these droplets. Additionally, the droplet size distribution may be controlled in emulsions, by controlling the conditions of emulsifications, such as stirring speed and stirrer geometry. As a result, a plurality of microcapsules is obtained with controlled average size and size distribution, wherein the oil phase is encapsulated and forms thereby the core of the microcapsules.

With respect to step h), the formation of the polymeric stabilizer is preferably initiated by adjusting the pH to a range of from 4.0 to 7.5, depending on the polymeric surfactant. For high methoxylated pectin, the optimal pH range is 6.5 ± 0.5, for an alginate, the optimal pH range is 7.0 ± 0.5 and for low methoxylated pectin and gum arabic, the optimal pH range is 4.5 ± 0.5.

The temperature is preferably maintained at room temperature for at least 1 h, and then increased to at least 60 °C, preferably at least 70 °C, more preferably at least 80 °C, but not more than 90 °C, for example 85 °C. Under these conditions, the formation of the shell is well controlled, meaning optimal stabilization of the interface is obtained.

The appropriate stirring speed and geometry of the mixer can be selected in order to obtain the desired average droplet size and droplet size distribution. It is a characteristic of the present invention that the polymeric stabilizer has sufficient interfacial activity and is able to promote the formation of dispersed oil droplets with desirable droplet size.

In a process according to the present invention, a one-liter vessel equipped with a turbine, or a cross-beam stirrer with pitched beam, such as a Mig stirrer, and having a stirrer diameter to reactor diameter of 0.6 to 0.8 may be used. Microcapsules can be formed in such reactor having a volume average size (d50) of 30 microns or less, more particularly 20 microns or less, at a stirring speed from about 100 to about 1200 rpm, more particularly from about 600 to 1000 rpm. Preferably, a Mig stirrer is used operating at a speed of 850 +/- 50 rpm. The person skilled in the art will however easily understand that such stirring conditions may change depending on the size of the reactor and of the batch size, on the exact geometry of the stirrer on the ratio of the diameter of the stirrer to the diameter of the reactor diameter ratios. For example, for a Mig stirrer with stirrer to reactor diameter ratio from 0.5 to 0.9 and slurry volumes ranging from 0.5 to 8 tons, the preferable agitation speed in the context of the present invention is from 150 rpm to 50 rpm.

In a particular embodiment of the present invention, the aminosilane to polymeric surfactant weight ratio in the emulsion is set within a range of from 0.1 to 1.1, more particularly from 0.2 to 0.9, still more particularly from 0.3 to 0.7, for example 0.35 or 0.65.

In a particular embodiment of the present invention, the shell material to oil weight ratio in the emulsion is set within a range from 0.01 to 0.5, more particularly from 0.025 to 0.4, even more particularly from 0.05 to 0.3.

Encapsulated compositions obtainable by the process mentioned hereinabove may be used as such or a polysaccharide, preferably a polysaccharide comprising beta (1 → 4) linked monosaccharide units, even more preferably a cellulose derivative, in particular selected form the group consisting of hydroxyethyl cellulose, hydroxpropylmethyl cellulose, cellulose acetate and carboxymethyl cellulose, preferably hydroxyethyl cellulose, may be added to the microcapsule shells formed in step h), as described in the above optional step i).

After formation of the microcapsules, the encapsulated composition is usually cooled to room temperature. Before, during or after cooling, the encapsulated composition may be further processed. Further processing may include treatment of the composition with anti-microbial preservatives, which preservatives are well known in the art. Further processing may also include the addition of a suspending aid, such as a hydrocolloid suspending aid to assist in the stable physical dispersion of the microcapsules and prevent any creaming or coalescence. Any additional adjuvants conventional in the art may also be added during further-processing.

In accordance with the process of the present invention, if desired, core-shell microcapsules may be further coated with a functional coating. A functional coating may entirely or only partially coat the microcapsule shell. Whether the functional coating is charged or uncharged, its primary purpose is to alter the surface properties of the microcapsule to achieve a desirable effect, such as to enhance the deposition of the microcapsule on a treated surface, such as a fabric, human skin or hair. Functional coatings may be post-coated to already formed microcapsules, or they may be physically incorporated into the microcapsule shell during shell formation. They may be attached to the shell by physical forces, physical interactions, such as hydrogen bonding, ionic interactions, hydrophobic interactions, electron transfer interactions, or they may be covalently bonded to the shell.

The at least one benefit agent can be at least one perfume ingredient. The at least one perfume ingredient can be selected from the group consisting of
ADOXAL^{™} (2,6,10-trimethylundec-9-enal); AGRUMEX^{™} (2-(tert-butyl)cyclohexyl acetate); ALDEHYDE C 10 DECYLIC (decanal); ALDEHYDE C 11 MOA (2-methyldecanal); ALDEHYDE C 11 UNDECYLENIC (undec-10-enal);
ALDEHYDE C 110 UNDECYLIC (undecanal); ALDEHYDE C 12 LAURIC (dodecanal); ALDEHYDE C 12 MNA PURE (2-methylundecanal); ALDEHYDE ISO C 11 ((E)-undec-9-enal); ALDEHYDE MANDARINE 10%/TEC ((E)-dodec-2-enal); ALLYL AMYL GLYCOLATE (allyl 2-(isopentyloxy)acetate); ALLYL CYCLOHEXYL PROPIONATE (allyl 3-cyclohexylpropanoate); ALLYL OENANTHATE (allyl heptanoate); AMBER CORE^{™} (1-((2-(tert-butyl)cyclohexyl)oxy)butan-2-ol); AMBERMAX^{™} (1,3,4,5,6,7-hexahydro-beta,1,1,5,5-pentamethyl-2H-2,4a-methanonaphthal-ene-8-ethanol); AMYL SALICYLATE (pentyl 2-hydroxybenzoate); APHERMATE (1-(3,3-dimethylcyclohexyl)ethyl formate);
BELAMBRE^{™} ((1R,2S,4R)-2'-isopropyl-1,7,7-trimethylspiro[bicyclo[2.2.1]heptane-2,4'-[1,3]dioxane]); BIGARYL (8-(sec-butyl)-5,6,7,8-tetrahydroquinoline); BOISAMBRENE FORTE^{™} ((ethoxymethoxy)cyclododecane); BOISIRIS^{™} ((1S,2R,5R)-2-ethoxy-2,6,6-trimethyl-9-methylenebicyclo[3.3.1]nonane); BORNYL ACETATE ((2S,4S)-1,7,7-trimethylbicyclo[2.2.1]heptan-2-yl acetate); BUTYL BUTYRO LACTATE (1-butoxy-1-oxopropan-2-yl butyrate); BUTYL CYCLOHEXYL ACETATE PARA (4-(tert-butyl)cyclohexyl acetate); CARYOPHYLLENE ((Z)-4,11,11-trimethyl-8-methylenebicyclo[7.2.0]undec-4-ene); CASHMERAN^{™} (1,1,2,3,3-pentamethyl-2,3,6,7-tetrahydro-1H-inden-4(5H)-one); CASSYRANE^{™} (5-tert-butyl-2-methyl-5-propyl-2H-furan); CITRAL ((E)-3,7-dimethylocta-2,6-dienal); CITRAL LEMAROME^{™} N ((E)-3,7-dimethylocta-2,6-dienal); CITRATHAL^{™} R ((Z)-1,1-diethoxy-3,7-dimethylocta-2,6-diene); CITRONELLAL (3,7-dimethyloct-6-enal);
CITRONELLOL (3,7-dimethyloct-6-en-1-ol); CITRONELLYL ACETATE (3,7-dimethyloct-6-en-1-yl acetate); CITRONELLYL FORMATE (3,7-dimethyloct-6-en-1-yl formate); CITRONELLYL NITRILE (3,7-dimethyloct-6-enenitrile);
CITRONELLYL PROPIONATE (3,7-dimethyloct-6-en-1-yl propionate); CLONAL (dodecanenitrile); CORANOL (4-cyclohexyl-2-methylbutan-2-ol); COSMONE^{™} ((Z)-3-methylcyclotetradec-5-enone); CYCLAMEN ALDEHYDE (3-(4-isopropylphenyl)-2-methylpropanal); CYCLOGALBANATE (allyl 2-(cyclohexyloxy)acetate); CYCLOHEXYL SALICYLATE (cyclohexyl 2-hydroxybenzoate); CYCLOMYRAL (8,8-dimethyl-1,2,3,4,5,6,7,8-octahydronaphthalene-2-carbaldehyde); DAMASCENONE ((E)-1-(2,6,6-trimethylcyclohexa-1,3-dien-1-yl)but-2-en-1-one); DAMASCONE ALPHA ((E)-1-(2,6,6-trimethylcyclohex-2-en-1-yl)but-2-en-1-one); DAMASCONE DELTA ((E)-1-(2,6,6-trimethylcyclohex-3-en-1-yl)but-2-en-1-one); DECENAL-4-TRANS ((E)-dec-4-enal); DELPHONE (2-pentylcyclopentanone); DIHYDRO ANETHOLE (propanedioic acid 1-(1-(3,3-dimethylcyclohexyl)ethyl) 3-ethyl ester);
DIHYDRO JASMONE (3-methyl-2-pentylcyclopent-2-enone); DIMETHYL BENZYL CARBINOL (2-methyl-1-phenylpropan-2-ol); DIMETHYL BENZYL CARBINYL ACETATE (2-methyl-1-phenylpropan-2-yl acetate); DIMETHYL BENZYL CARBINYL BUTYRATE (2-methyl-1-phenylpropan-2-yl butyrate);
DIMETHYL OCTENONE (4,7-dimethyloct-6-en-3-one); DIMETOL (2,6-dimethylheptan-2-ol); DIPENTENE (1-methyl-4-(prop-1-en-2-yl)cyclohex-1-ene); DUPICAL^{™} ((E)-4-((3aS,7aS)-hexahydro-1H-4,7-methanoinden-5(6H)-ylidene)butanal); EBANOL^{™} ((E)-3-methyl-5-(2,2,3-trimethylcyclopent-3-en-1-yl)pent-4-en-2-ol); ETHYL CAPROATE (ethyl hexanoate); ETHYL CAPRYLATE (ethyl octanoate); ETHYL LINALOOL ((E)-3,7-dimethylnona-1,6-dien-3-ol);
ETHYL LINALYL ACETATE ((Z)-3,7-dimethylnona-1,6-dien-3-yl acetate); ETHYL OENANTHATE (ethyl heptanoate); ETHYL SAFRANATE (ethyl 2,6,6-trimethylcyclohexa-1,3-diene-1-carboxylate); EUCALYPTOL ((1s,4s)-1,3,3-trimethyl-2-oxabicyclo[2.2.2]octane); FENCHYL ACETATE ((2S)-1,3,3-trimethylbicyclo[2.2.1]heptan-2-yl acetate); FENCHYL ALCOHOL ((1S,2R,4R)-1,3,3-trimethylbicyclo[2.2.1]heptan-2-ol); FlXOLIDE^{™} (1-(3,5,5,6,8,8-hexamethyl-5,6,7,8-tetrahydronaphthalen-2-yl)ethanone); FLORALOZONE^{™} (3-(4-ethylphenyl)-2,2-dimethylpropanal); FLORHYDRAL (3-(3-isopropylphenyl)butanal); FLOROCYCLENE^{™} ((3aR,6S,7aS)-3a,4,5,6,7,7a-hexahydro-1H-4,7-methanoinden-6-yl propionate); FLOROPAL^{™} (2,4,6-trimethyl-4-phenyl-1,3-dioxane); FRESKOMENTHE^{™} (2-(sec-butyl)cyclohexanone); FRUITATE ((3aS,4S,7R,7aS)-ethyl octahydro-1H-4,7-methanoindene-3a-carboxylate); FRUTONILE (2-methyldecanenitrile);
GALBANONE^{™} PURE (1-(3,3-dimethylcyclohex-1-en-1-yl)pent-4-en-1-one);
GARDOCYCLENE^{™} ((3aR,6S,7aS)-3a,4,5,6,7,7a-hexahydro-1H-4,7-methanoinden-6-yl isobutyrate); GERANIOL ((E)-3,7-dimethylocta-2,6-dien-1-ol); GERANYL ACETATE SYNTHETIC ((E)-3,7-dimethylocta-2,6-dien-1-yl acetate); GERANYL ISOBUTYRATE ((E)-3,7-dimethylocta-2,6-dien-1-yl isobutyrate); GIVESCONE^{™} (ethyl 2-ethyl-6,6-dimethylcyclohex-2-enecarboxylate); HABANOLIDE^{™} ((E)-oxacyclohexadec-12-en-2-one);
HEDIONE^{™} (methyl 3-oxo-2-pentylcyclopentaneacetate); HERBANATE^{™} ((2S)-ethyl 3-isopropylbicyclo[2.2.1]hept-5-ene-2-carboxylate); HEXENYL-3-CIS BUTYRATE ((Z)-hex-3-en-1-yl butyrate); HEXYL CINNAMIC ALDEHYDE ((E)-2-benzylideneoctanal); HEXYL ISOBUTYRATE (hexyl isobutyrate); HEXYL SALICYLATE (hexyl 2-hydroxybenzoate); INDOFLOR^{™} (4,4a,5,9b-tetrahydroindeno[1,2-d][1,3]dioxine); IONONE BETA ((E)-4-(2,6,6-trimethylcyclohex-1-en-1-yl)but-3-en-2-one); IRISONE ALPHA ((E)-4-(2,6,6-trimethylcyclohex-2-en-1-yl)but-3-en-2-one); IRONE ALPHA ((E)-4-(2,5,6,6-tetramethylcyclohex-2-en-1-yl)but-3-en-2-one); ISO E SUPER^{™} (1-(2,3,8,8-tetramethyl-1,2,3,4,5,6,7,8-octahydronaphthalen-2-yl)ethanone);
ISOCYCLOCITRAL (2,4,6-trimethylcyclohex-3-enecarbaldehyde); ISONONYL ACETATE (3,5,5-trimethylhexyl acetate); ISOPROPYL METHYL-2-BUTYRATE (isopropyl 2-methyl butanoate); ISORALDEINE^{™} 70 ((E)-3-methyl-4-(2,6,6-trimethylcyclohex-2-en-1-yl)but-3-en-2-one); JASMACYCLENE^{™} ((3aR,6S,7aS)-3a,4,5,6,7,7a-hexahydro-1H-4,7-methanoinden-6-yl acetate);
JASMONE CIS ((Z)-3-methyl-2-(pent-2-en-1-yl)cyclopent-2-enone);
KARANAL^{™} (5-(sec-butyl)-2-(2,4-dimethylcyclohex-3-en-1-yl)-5-methyl-1,3-dioxane); KOAVONE ((Z)-3,4,5,6,6-pentamethylhept-3-en-2-one); LEAF ACETAL ((Z)-1-(1-ethoxyethoxy)hex-3-ene); LEMONILE^{™} ((2E,6Z)-3,7-dimethylnona-2,6-dienenitrile); LIFFAROME^{™} GIV ((Z)-hex-3-en-1-yl methyl carbonate); LILIAL^{™} (3-(4-(tert-butyl)phenyl)-2-methylpropanal); LINALOOL (3,7-dimethylocta-1,6-dien-3-ol); LINALYL ACETATE (3,7-dimethylocta-1,6-dien-3-yl acetate); MAHONIAL^{™} ((4E)-9-hydroxy-5,9-dimethyl-4-decenal);
MALTYL ISOBUTYRATE (2-methyl-4-oxo-4H-pyran-3-yl isobutyrate);
MANZANATE (ethyl 2-methylpentanoate); MELONAL^{™} (2,6-dimethylhept-5-enal); MENTHOL (2-isopropyl-5-methylcyclohexanol); MENTHONE (2-isopropyl-5-methylcyclohexanone); METHYL CEDRYL KETONE (1-((1S,8aS)-1,4,4,6-tetramethyl-2,3,3a,4,5,8-hexahydro-1H-5,8a-methanoazulen-7-yl)ethanone);
METHYL NONYL KETONE EXTRA (undecan-2-one); METHYL OCTYNE CARBONATE (methyl non-2-ynoate); METHYL PAMPLEMOUSSE (6,6-dimethoxy-2,5,5-trimethylhex-2-ene); MYRALDENE (4-(4-methylpent-3-en-1-yl)cyclohex-3-enecarbaldehyde); NECTARYL (2-(2-(4-methylcyclohex-3-en-1-yl)propyl)cyclopentanone); NEOBERGAMATE^{™} FORTE (2-methyl-6-methyleneoct-7-en-2-yl acetate); NEOFOLIONE^{™} ((E)-methyl non-2-enoate);
NEROLIDYLE^{™} ((Z)-3,7,11-trimethyldodeca-1,6,10-trien-3-yl acetate); NERYL ACETATE HC ((Z)-3,7-dimethylocta-2,6-dien-1-yl acetate); NONADYL (6,8-dimethylnonan-2-ol); NONENAL-6-CIS ((Z)-non-6-enal); NYMPHEAL^{™} (3-(4-isobutyl-2-methylphenyl)propanal); ORIVONE^{™} (4-(tert-pentyl) cyclohexanone); PARADISAMIDE^{™} (2-ethyl-N-methyl-N-(m-tolyl)butanamide); PELARGENE (2-methyl-4-methylene-6-phenyltetrahydro-2H-pyran); PEONILE^{™} (2-cyclohexylidene-2-phenylacetonitrile); PETALIA^{™} (2-cyclohexylidene-2-(o-tolyl)acetonitrile); PIVAROSE^{™} (2,2-dimethyl-2-pheylethyl propanoate); PRECYCLEMONE^{™} B (1-methyl-4-(4-methylpent-3-en-1-yl)cyclohex-3-enecarbaldehyde); PYRALONE^{™} (6-(sec-butyl)quinoline);
RADJANOL^{™} SUPER ((E)-2-ethyl-4-(2,2,3-trimethylcyclopent-3-en-1-yl)but-2-en-1-ol); RASPBERRY KETONE (N112) (4-(4-hydroxyphenyl)butan-2-one);
RHUBAFURANE^{™} (2,2,5-trimethyl-5-pentylcyclopentanone); ROSACETOL (2,2,2-trichloro-1-phenylethyl acetate); ROSALVA (dec-9-en-1-ol); ROSYFOLIA ((1-methyl-2-(5-methylhex-4-en-2-yl)cyclopropyl)-methanol); ROSYRANE^{™} SUPER (4-methylene-2-phenyltetrahydro-2H-pyran); SERENOLIDE (2-(1-(3,3-dimethylcyclohexyl)ethoxy)-2-methylpropyl cyclopropanecarboxylate);
SILVIAL^{™} (3-(4-isobutylphenyl)-2-methylpropanal); SPIROGALBANONE^{™} (1-(spiro[4.5]dec-6-en-7-yl)pent-4-en-1-one); STEMONE^{™} ((E)-5-methylheptan-3-one oxime); SUPER MUGUET^{™} ((E)-6-ethyl-3-methyloct-6-en-1-ol);
SYLKOLIDE^{™} ((E)-2-((3,5-dimethylhex-3-en-2-yl)oxy)-2-methylpropyl cyclopropanecarboxylate); TERPINENE GAMMA (1-methyl-4-propan-2-ylcyclohexa-1,4-diene); TERPINOLENE (1-methyl-4-(propan-2-ylidene)cyclohex-1-ene); TERPINYL ACETATE (2-(4-methylcyclohex-3-en-1-yl)propan-2-yl acetate); TETRAHYDRO LINALOOL (3,7-dimethyloctan-3-ol);
TETRAHYDRO MYRCENOL (2,6-dimethyloctan-2-ol); THIBETOLIDE (oxacyclohexadecan-2-one); TRIDECENE-2-NITRILE ((E)-tridec-2-enenitrile);
UNDECAVERTOL ((E)-4-methyldec-3-en-5-ol); VELOUTONE^{™} (2,2,5-trimethyl-5-pentylcyclopentanone); VIRIDINE^{™} ((2,2-dimethoxyethyl)benzene);
ZINARINE^{™} (2-(2,4-dimethylcyclohexyl)pyridine); and mixtures thereof.

A comprehensive list of perfume ingredients that may be encapsulated in accordance with the present invention can be found in the perfumery literature, for example *"*Perfume & Flavor Chemicals", S. Arctander, Allured Publishing, 2000.

The at least one benefit agent can also be a cosmetic ingredient. Preferably, the cosmetic ingredients have a calculated octanol/water partition coefficient (ClogP) of 1.5 or more, more preferably 3 or more. Alternatively preferred, the ClogPof the cosmetic ingredient is from 2 to 7.

Particularly useful cosmetic ingredients may be selected from the group consisting of emollients, smoothening actives, hydrating actives, soothing and relaxing actives, decorative actives, anti-aging actives, draining actives, remodelling actives, skin levelling actives, preservatives, anti-oxidant actives, antibacterial or bacteriostatic actives, cleansing actives, lubricating actives, structuring actives, hair conditioning actives, whitening actives, texturing actives, softening actives, anti-dandruff actives and exfoliating actives.

Particularly useful cosmetic ingredients include, but are not limited to, hydrophobic polymers, such as alkyldimethylsiloxanes, polymethyl silsesquioxanes, polyethylene, polyisobutylene, styrene-ethylene-styrene and styrene-butylene-styrene block copolymers, mineral oils, such as hydrogenated isoparaffins, silicone oils, vegetable oils, such as argan oil, jojoba oil, aloe vera oil, fatty acids and fatty alcohols and their esters, glycolipides, phospholipides, sphingolipides, such as ceramides, sterols and steroids, terpenes, sesquiterpenes, triterpenes and their derivatives, essential oils, such as arnica oil, artemisia oil, bark tree oil, birch leaf oil, calendula oil, cinnamon oil, echinacea oil, eucalyptus oil, ginseng oil, jujube oil, helianthus oil, jasmine oil, lavender oil, lotus seed oil, perilla oil, rosmary oil, sandal wood oil, tea tree oil, thyme oil, valerian oil, wormwood oil, ylang ylang oil and yucca oil.

The resultant encapsulated composition, presented in the form of a slurry of microcapsules suspended in an aqueous suspending medium may be incorporated as such in a consumer product base. If desired, however, the slurry may be dried to present the encapsulated composition in dry powder form. Drying of a slurry of microcapsules is conventional, and may be carried out according techniques known in the art, such as spray-drying, evaporation, lyophilization or use of a desiccant. Typically, as is conventional in the art, dried microcapsules will be dispersed or suspended in a suitable powder, such as powdered silica, which can act as a bulking agent or flow aid. Such suitable powder may be added to the encapsulated composition before, during or after the drying step.

A further aspect of the present invention relates to an encapsulated composition obtainable any of the methods described herein above.

Yet another aspect of the present invention relates to a use of an encapsulated composition as described herein above to enhance the performance of a benefit agent in a consumer product.

The present invention also relates to a consumer product comprising an encapsulated composition as described herein above. The consumer product is preferably selected from the group consisting of fabric care detergents and conditioners, hair care conditioners, shampoos, heavy duty liquid detergents, hard surface cleaners, detergent powders, soaps, shower gels and skin care products.

Encapsulated compositions according to the present invention are particularly useful when employed as perfume delivery vehicles in consumer goods that require, for delivering optimal perfumery benefits, that the microcapsules adhere well to a substrate on which they are applied. Such consumer goods include hair shampoos and conditioners, as well as textile-treatment products, such as laundry detergents and conditioners.

A further aspect of the present invention relates to a polymeric stabilizer formed by combination of a polymeric surfactant with at least one aminosilane, as described herein above, wherein the aminosilane is a bipodal aminosilane. The polymeric surfactant comprises a polysaccharide comprising carboxylic acid groups and is in particular a polymeric surfactant as described herein above.

Yet another aspect of the present invention relates to a use of a polymeric stabilizer as described herein above in the encapsulation of a benefit agent. The polymeric stabilizer stabilizes the oil/water interfaces and, thereby, provides a template for the preparation of encapsulated perfume and/or cosmetic compositions.

The present disclosure also relates to a method for enhancing the performance of a benefit agent in a consumer product by adding an encapsulated composition according to the present invention.

Furthermore, the present disclosure refers to a method of encapsulating a benefit agent, wherein the polymeric stabilizer as described herein above stabilizes and encapsulates the oil droplets of the oil in water emulsion, and wherein the oil phase comprises the at least one benefit agent.

Particular features and further advantages of the present invention become apparent from the following examples.

### Example 1 - Formation of microcapsules having first shell comprising pectin as polymeric surfactant

The microcapsules have been obtained by performing the steps of:
a) Preparing a core composition by admixing 0.7 g of bipodal aminosilane (bis(3-triethoxysilylpropyl)amine) and 25 g of fragrance composition;
b) Emulsifying the core composition obtained in step a) in a mixture of 1.4 g low methoxylated grade pectin (of type APA 220, ex Roeper) in 68.6 g of water by using a 300 ml reactor and a cross-beam stirrer with pitched beam operating at a stirring speed of 850 rpm at a temperature of 25 +/- 2 °C for 10 min;
c) Adjusting the pH of the continuous phase of the emulsion to 6.5 +/- 0.5 with a 10 % sodium hydroxide solution in water and maintaining the system at a temperature of 25 +/- 2 °C for 1 h while maintaining stirring as in step b);
d) Increasing progressively the temperature to 85 °C over 2.5 h and maintaining the temperature at 85 °C for 1 h, while maintaining stirring as in steps b) and c) to complete the formation of core-shell capsules;
e) Letting the slurry of core-shell capsules obtained in step d) cool to room temperature.

The solid content of each of the slurries was measured by using a thermo-balance operating at 120 °C. The solid content, expressed as weight percentage of the initial slurry deposited on the balance was taken at the point where the drying-induced rate of weight change had dropped below 0.1 %/min. The ratio of the measured solid content to the theoretical solid content calculated based on the weight of perfume and encapsulating materials involved is taken as a measurement of encapsulation yield, expressed in wt.-%.

The solid content of the slurry obtained was 5 wt.-%, the volume average size (d50) of the capsules was 17 ± 3 µm and the encapsulation efficiency of 16 %.

### Example 2 - Formation of microcapsules having first shell comprising pectin as polymeric surfactant and isocyanate

The microcapsules have been obtained by performing the steps of:
a) Preparing a core composition by admixing 0.7 g of bipodal aminosilane (bis(3-triethoxysilylpropyl)amine) and 0.4 g of Takenate D-110N (ex Mitsui) and 25 g of fragrance composition;
b) Emulsifying the core composition obtained in step a) in a mixture of 1.4 g low methoxylated grade pectin (of type APA 220, ex Roeper) in 68.6 g of water by using a 300 ml reactor and a cross-beam stirrer with pitched beam operating at a stirring speed of 850 rpm at a temperature of 25 +/- 2 °C for 10 min;
c) Adjusting the pH of the continuous phase of the emulsion to 4.5 +/- 0.5 with a 10 % sodium hydroxide solution in water and maintaining the system at a temperature of 25 +/- 2 °C for 1 h while maintaining stirring as in step b);
d) Increasing progressively the temperature to 85 °C over 2.5 h and maintaining the temperature at 85 °C for 1 h, while maintaining stirring as in steps b) and c) to complete the formation of core-shell capsules;
e) Letting the slurry of core-shell capsules obtained in step d) cool to room temperature.

The solid content of the slurry obtained was 27 wt.-%, the volume average size (d50) of the capsules was 15 µm and the encapsulation efficiency of 95 +/- 5 %.

### Example 3 - Formation of microcapsules comprising ZeMac E400 and 2-hydroxyethyl cellulose

The microcapsules have been obtained by performing the steps of (Example 3.1):
a) Preparing a core composition by admixing 1 g of bipodal aminosilane (bis(3-triethoxysilylpropyl)amine), 0.4 g of Takenate D-110N (ex Mitsui) and 35.5 g of fragrance composition;
b) Emulsifying the core composition obtained in step a) in a mixture of 1.5 g ZeMac E400 (ex Vertellus) in 51.1 g of water by using a 300 ml reactor and a cross-beam stirrer with pitched beam operating at a stirring speed of 800 rpm at a temperature of 25 +/- 2 °C for 10 min;
c) Adjusting the pH of the continuous phase of the emulsion to 4.4 +/- 0.5 with a 10 % sodium hydroxide solution in water and maintaining the system at a temperature of 25 +/- 2 °C for 1 h while maintaining stirring as in step b);
d) Increasing progressively the temperature to 85 °C over 2.5 h and maintaining the temperature at 85 °C for 1 h, while maintaining stirring as in steps b) and c) to complete the formation of core-shell capsules;
e) Adding 37.5 g of a 7.2 wt.-% of a solution of 2-hydroxyethyl cellulose in water and keeping stirring for 1 h at 85 °C;
f) Adding 0.8 g of a solution of citric acid diluted at 30 % in water and keeping stirring for 1 h at 85 °C;
g) Letting the slurry of core-shell capsules obtained in step f) cool to room temperature.

The solid content of the slurry obtained was 32 wt.-%, the volume average size (d50) of the capsules was 15 µm and the encapsulation efficiency of 95 +/-5%.

For application in hair care conditioner (Example 3.2), 14.4 g of a 4 % Polyquaternium 10 (Ucare JR400, ex Dow Chemicals) in water was added in the slurry after cooling.

### Example 4 - Formation of microcapsules comprising low methoxylated grade pectin and 2-hydroxyethyl cellulose

The microcapsules have been obtained by performing the steps of (Example 4.1):
a) Preparing a core composition by admixing 0.57 g of bipodal aminosilane (bis(3-triethoxysilylpropyl)amine), 0.8 g of Takenate D-110N (ex Mitsui) and 20 g of fragrance composition;
b) Emulsifying the core composition obtained in step a) in a mixture of 1.1 g low methoxylated grade pectin (of type APA 220, ex Roeper) in 54.9 g of water by using a 300 ml reactor and a cross-beam stirrer with pitched beam operating at a stirring speed of 850 rpm at a temperature of 25 +/- 2 °C for 10 min;
c) Adjusting the pH of the continuous phase of the emulsion to 4.5 +/- 0.5 with a 10% sodium hydroxide solution in water and maintaining the system at a temperature of 25 +/- 2 °C for 1 h while maintaining stirring as in step b);
d) Increasing progressively the temperature to 85 °C over 2.5 h and maintaining the temperature at 85 °C for 1 h, while maintaining stirring as in steps b) and c) to complete the formation of core-shell capsules;
e) Adding 21.4 g of 2-hydroxyethyl cellulose diluted at 7.2 % in water and continue stirring for 1 h at 85 °C;
f) Adding 0.8 g of a solution of citric acid diluted at 30 % in water and continue stirring for 1 h at 85°C;
g) Letting the slurry of core-shell capsules obtained in step f) cool to room temperature.

The solid content of the slurry obtained was 30 wt.-%, the volume average size (d50) of the capsules was 20 µm and the encapsulation efficiency of 90 +/- 5 %.

In Example 4.2, the microcapsules have been obtained as for Example 4.1, but the 2-hydroxyethyl cellulose has been added as a powder to the system in step e). In this case, the amount of 2-hydroxyethyl cellulose was 1.5 g. The solid content of this slurry obtained was 30 %, the volume average size (d50) of the capsules was 17 +/- 3 µm and the encapsulation efficiency 90 +/- 5 %.

For application in hair care conditioner (Example 4.3), 14.4 g of a 4 % Polyquaternium 10 (Ucare JR400, ex Dow Chemicals) in water was added in the slurry after cooling.

In a further example, the microcapsules have been obtained by performing the steps of (Example 4.4):
a) Preparing a core composition by admixing 0.66 g of bipodal aminosilane (bis(3-triethoxysilylpropyl)amine), 0.47 g of Takenate D-110N (ex Mitsui) and 38.5 g of fragrance composition;
b) Emulsifying the core composition obtained in step a) in a mixture of 1.35 g low methoxylated grade pectin (of type APA 220, ex Roeper) in 66.2 g of water by using a 300 ml reactor and a cross-beam stirrer with pitched beam operating at a stirring speed of 800 rpm at a temperature of 25 +/- 2 °C for 10 min;
c) Adjusting the pH of the continuous phase of the emulsion to 6 +/- 1 with a 10% sodium hydroxide solution in water and maintaining the system at a temperature of 25 +/- 2 °C for 1 h while maintaining stirring as in step b);
d) Increasing progressively the temperature to 85 °C over 2.5 h and maintaining the temperature at 85 °C for 1 h, while maintaining stirring as in steps b) and c) to complete the formation of core-shell capsules;
e) Adding 1.8 g of 2-hydroxyethyl cellulose and continue stirring for 30 min at 85 °C;
f) Adding 0.8 g of a solution of citric acid diluted at 30 % in water and continue stirring for 1 h at 85°C;
g) Letting the slurry of core-shell capsules obtained in step f) cool to room temperature.

The solid content of the slurry obtained was 40 wt.-%, the volume average size (d50) of the capsules was 20 +/- 5 µm and the encapsulation efficiency of 90 +/- 5 %.

In Example 4.5, the microcapsules have been obtained as for Example 4.4, but the solution of citric acid has been replaced with benzene-1,3,5-tricarboxylic acid in step f). In this case, the amount of benzene-1,3,5-tricarboxylic acid was 0.3 g. The solid content of this slurry obtained was 40 wt.-%, the volume average size (d50) of the capsules was 20 +/- 5 µm and the encapsulation efficiency of 95 +/- 5 %.

In Example 4.6, the microcapsules have been obtained as for Example 4.4, but the solution of citric acid has been replaced with 2,5-furandicarboxylic acid in step f). In this case, the amount of 2,5-furandicarboxylic acid was 0.15 g. The solid content of this slurry obtained was 40 wt.-%, the volume average size (d50) of the capsules was 20 +/- 5 µm and the encapsulation efficiency of 95 +/-5%.

### Example 5 - Formation of microcapsules comprising high methoxylated pectin and 2-hydroxyethyl cellulose

The microcapsules have been obtained by performing the steps of (Example 5.1):
a) Preparing a core composition by admixing 0.57 g of bipodal aminosilane (bis(3-triethoxysilylpropyl)amine), 0.8 g of Takenate D-110N (ex Mitsui) and 20 g of fragrance composition;
b) Emulsifying the core composition obtained in step a) in a mixture of 1.1 g high methoxylated grade pectin (of type APA 104, ex Roeper) in 54.9 g of water by using a 300 ml reactor and a cross-beam stirrer with pitched beam operating at a stirring speed of 850 rpm at a temperature of 25 +/- 2 °C for 10 min;
c) Adjusting the pH of the continuous phase of the emulsion to 6.5 +/- 0.5 with a 10% sodium hydroxide solution in water and maintaining the system at a temperature of 25 +/- 2 °C for 1 h while maintaining stirring as in step b);
d) Increasing progressively the temperature to 85 °C over 2.5 h and maintaining the temperature at 85 °C for 1 h, while maintaining stirring as in steps b) and c) to complete the formation of core-shell capsules;
e) Adding 21.4 g of 2-hydroxyethyl cellulose diluted at 7.2 % in water and continue stirring for 1 h at 85 °C;
f) Adding 0.8 g of a solution of citric acid diluted at 30 % in water and continue stirring for 1 h at 85 °C;
g) Letting the slurry of core-shell capsules obtained in step f) cool to room temperature.

The solid content of the slurry obtained was 30 wt.-%, the volume average size (d50) of the capsules was 20 µm and the encapsulation efficiency of 90 +/-5%.

In Example 5.2, the microcapsules have been obtained as for Example 5.1, but the 2-hydroxyethyl cellulose has been added as a powder to the system in step e). In this case, the amount of 2-hydroxyethyl cellulose was 1.5 g. The solid content of this slurry obtained was 30 wt.-%, the volume average size (d50) of the capsules was 17 +/- 3 µm and the encapsulation efficiency of 90 +/- 5 %.

For application in hair care conditioner (Example 5.3), 14.4 g of a 4 % solution of Polyquaternium 10 (Ucare JR400, ex Dow Chemicals) in water was added in the slurry after cooling.

In a further example, the microcapsules have been obtained by performing the steps of (Example 5.4):
a) Preparing a core composition by admixing 0.66 g of bipodal aminosilane (bis(3-triethoxysilylpropyl)amine), 0.48 g of Takenate D-110N (ex Mitsui) and 38.5 g of fragrance composition;
b) Emulsifying the core composition obtained in step a) in a mixture of 1.35 g high methoxylated grade pectin (of type APA 104, ex Roeper) in 66.2 g of water by using a 300 ml reactor and a cross-beam stirrer with pitched beam operating at a stirring speed of 800 rpm at a temperature of 25 +/- 2 °C for 10 min;
c) Adjusting the pH of the continuous phase of the emulsion to 6.5 +/- 0.5 with a 10% sodium hydroxide solution in water and maintaining the system at a temperature of 25 +/- 2 °C for 1 h while maintaining stirring as in step b);
d) Increasing progressively the temperature to 85 °C over 2.5 h and maintaining the temperature at 85 °C for 1 h, while maintaining stirring as in steps b) and c) to complete the formation of core-shell capsules;
e) Adding 1.8 g of 2-hydroxyethyl cellulose and continue stirring for 30min at 85 °C;
f) Adding 0.8 g of a solution of citric acid diluted at 30 % in water and continue stirring for 1 h at 85 °C;
g) Letting the slurry of core-shell capsules obtained in step f) cool to room temperature.

The solid content of the slurry obtained was 40 wt.-%, the volume average size (d50) of the capsules was 20+/- 5 µm and the encapsulation efficiency of 90 +/- 5 %.

In Example 5.5, the microcapsules have been obtained as for Example 5.4, but the solution of citric acid has been replaced with benzene-1,3,5-tricarboxylic acid in step f). In this case, the amount of benzene-1,3,5-tricarboxylic acid was 0.3 g. The solid content of this slurry obtained was 40 wt.-%, the volume average size (d50) of the capsules was 20 +/- 5 µm and the encapsulation efficiency of 95 +/- 5 %.

In Example 5.6, the microcapsules have been obtained as for Example 5.4, but the solution of citric acid has been replaced with 2,5-furandicarboxylic acid in step f). In this case, the amount of 2,5-furandicarboxylic acid was 0.15 g. The solid content of this slurry obtained was 40 wt.-%, the volume average size (d50) of the capsules was 20 +/- 5 µm and the encapsulation efficiency of 95 +/- 5 %.

### Example 6 - Formation of microcapsules comprising gum arabic and 2-hydroxyethyl cellulose

The microcapsules have been obtained by performing the steps of (Example 6.1):
a) Preparing a core composition by admixing 1 g of bipodal aminosilane (bis(3-triethoxysilylpropyl)amine), 1 g of Takenate D-110 (ex Mitsui), 2.25 g of Bayhydur XP 2547 (ex Covestro) and 35.5 g of fragrance composition;
b) Emulsifying the core composition obtained in step a) in a mixture of 5 g of gum arabic Senegal and 48 g of water by using a 300 ml reactor and a cross-beam stirrer with pitched beam operating at a stirring speed of 300 rpm at a temperature of 25 +/- 2 °C for 10 min;
c) Adjusting the pH of the continuous phase of the emulsion to 4.4 +/- 0.5 with a 10 % formic acid solution in water and maintaining the system at a temperature of 25 +/- 2 °C for 1 h while increasing the stirring to 700 rpm;
d) Increasing progressively the temperature to 85 °C over 2.5 h and maintaining the temperature at 85 °C for 1 h, while maintaining stirring as in step c) to complete the formation of core-shell capsules;
e) Adding 37.1 g of a 1 wt.-% solution of 2-hydroxyethyl cellulose in water and continue stirring for 1 h at 85 C°;
f) Letting the slurry of core-shell capsules obtained in step f) cool to room temperature.

The solid content of the slurry obtained in step g) was 33.9 wt.-%, the volume average size (d50) of the capsules was 11.7 µm and the encapsulation efficiency of 98 wt.-%.

In Example 6.2, the process of Example 6.1 was repeated, but with gum tragacanth instead of gum arabic Senegal. Additionally, the quantity of gum tragacanth was half of the quantity of gum arabic Senegal, due to the high viscosity of gum tragacanth.

### Example 7 - Formation of microcapsules comprising alginate and 2-hydroxyethyl cellulose

The microcapsules have been obtained by performing the steps of (Example 7.1):
a) Preparing a core composition by admixing 0.5 g of bipodal aminosilane (bis(3-triethoxysilylpropyl)amine), 0.04 g of Takenate D-110N (ex Mitsui) and 17.75 g of fragrance composition;
b) Emulsifying the core composition obtained in step a) in a 26.3 g of an aqueous solution containing 2 wt.-% of alginate (Scogin XL, ex FMC corporation) and 0.1 wt.-% of Tween 85 by using a 100 ml reactor and a cross-beam stirrer with pitched beam operating at a stirring speed of 850 rpm at a temperature of 25 +/- 2 °C for 10 min;
c) Adjusting the pH of the continuous phase of the emulsion to 5.0 +/- 0.5 with a 10 % formic acid solution in water and maintaining the system at a temperature of 35 +/- 2 °C for 1 h while increasing the stirring to 700 rpm;
d) Increasing progressively the temperature to 85 °C over 2.5 h;
e) Adding 37.5 g of a 7.2 wt.-% solution of 2-hydroxyethyl cellulose in water and maintaining the temperature at 85 °C for 1 h, while maintaining stirring as in step c);
f) Adding 0.8 g of Bayhydur XP2547 and continue to stir for 1 h at 85 °C in order to complete the formation of core-shell capsules;
g) Letting the slurry of core-shell capsules obtained in step f) cool to room temperature.

The solid content of the slurry obtained in step g) was 22 wt.-%, the volume average size (d50) of the capsules was 40 µm and the encapsulation efficiency of 92 %.

In Example 7.2, microcapsules were obtained under the same conditions as Example 7.1, but Takenate D-110N was replaced by isophtaldehyde. The solid content of the slurry obtained was 26 wt.-%, the volume average size (d50) of the capsules was 40 µm and the encapsulation efficiency of 100 %.

### Example 8 - Aminoplast Capsules (Comparative Example)

Aminoplast microcapsules were obtained by performing the method disclosed in WO 2016/207187 A1, Example 2b.

The solid content of the slurry obtained was 45 %, the volume average size (d50) of the capsules was 20 µm and the encapsulation efficiency of 100 %.

### Example 9 - Assessment of leakage of microcapsules in a laundry care conditioner base

The base was an unperfumed commercial proprietary laundry care conditioner base. For each assessment 1 wt.-% of slurry was dispersed in the base under stirring with a paddle mixer. The encapsulated core composition comprised additionally 0.02 wt.-% of Hostasol^{®} Yellow 3G (Clariant) as fluorescent dye. The samples were then stored for 8 weeks at 37 °C. The leakage from the capsules was assessed visually by fluorescent light microscopy, operating at 488 nm excitation light wavelength and 515 nm emission light wavelength, according to the following scale:
- Poor stability: Collapsed microcapsules and fluorescent droplets are visible;
- Average stability: Partially collapsed microcapsules coexist with fluorescent droplets;
- Good stability: All capsules are still full of fluorescent core composition and no fluorescent droplets are visible.

Representative leakage values are given in Table 1, herein below.

### Example 10 - Assessment of fragrance release performance

The release performance of the microcapsule slurries was measured by using a texture analyzer (TA XT PLUS, ex TA instruments). 300 microliters of undiluted slurry were deposited on the surface of filter paper in three successive applications of 100 microliters and left to dry overnight. Then, the lower surface of a mechanical sensor probe, consisting of a flat metal cylinder having a diameter of 12.5 micrometer, was applied on the deposited microcapsules with a penetration velocity of 0.01 mm/s.

As the probe penetrates the bed of microcapsules deposited on the filter paper, it experiences a back elastic force which is proportional to the elastic bending modulus of the microcapsules, which is inversely proportional to the release performance of the microcapsules. The value of the measured force at the 50 % deformation of the microcapsule bed is taken as a measurement of the release performance of the microcapsules. The displacement corresponding to 50 % deformation point is determined as the half way point between the displacement point where the first contact with the microcapsules occurs, which is marked by the onset of a back force and the point where the probe motion is stopped by the filter paper.

**Table 1: Perfume leakage in water/ethanol/cyclohexane and force at 50% deformation for selected examples**

| Example | Leakage in laundry care conditioner [visual scale, see Example 9] | Force at 50% deformation [N] |
|---|---|---|
| Example 1 | Average | n.a. * |
| Example 2 | Average to good | 2.6 |
| Example 3.1 | Good | 3.0 |
| Example 4.1 | Good | 5.2 |
| Example 4.5 | Good | 2.6 |
| Example 4.6 | Good | 2.7 |
| Example 5.1 | Good | 4.0 |
| Example 5.5 | Good | n.a. |
| Example 5.6 | Good | n.a. |
| Example 6.1 | Good | 4.0 |
| Example 6.2 | Poor | < 1 |
| Example 7.1 | Average | < 1 |
| Example 8 | Good | 6.5 |

| | | |
|---|---|---|
| * Sample too viscous. | | |

It may be concluded from these results that the capsules according to the present invention have a stability in laundry care conditioner base that is similar to the one of conventional capsules based on aminoplast and polyurea resins.

### Example 11 - Comparison of olfactive performance of new and conventional microcapsules

The olfactive performance of the microcapsule was assessed by a panel of 4 experts who rated the odor intensity on a scale of 1-5 (1 = barely noticeable, 2 = weak, 3 = medium, 4 = strong and 5 = very strong). When relevant, qualitative comments on the perceived odor direction were recorded.

For application in laundry care, the samples were evaluated in an unperfumed commercial proprietary fabric care softener. The aforementioned microcapsule slurries were added to a fabric care conditioner composition under gentle stirring with a paddle mixer, so that the level of slurry in the fabric care conditioner base was 1.5 wt.-% referred to the total weight of the hair care conditioner base. 35 g of fabric care conditioner was put in a front-loaded wash machine containing 720 g of terry toweling and operating with a total volume of 15 l water. The *"out-of-the-wash machine"* odor intensity was assessed on wet toweling within 5 min after having removed the toweling from the machine. The pre-rub olfactive evaluation was performed after drying the toweling for 24 h at room temperature. The post-rub evaluation was performed by gently rubbing one part of the toweling.

For application in hair care conditioner, the samples were evaluated in a unperfumed hair care conditioner. The aforementioned microcapsule slurries were added to a hair care conditioner composition under gentle stirring with a paddle mixer, so that the level of slurry in the hair care conditioner base was 1 wt.-% referred to the total weight of the hair care conditioner base. 1.5 g of hair care conditioner was applied on 15 g swatches humidified with 12 g water. The swatches were submitted to a massage, left to stand for 1 min and then rinsed 30 seconds under running tap water at 37 °C at a flow rate of 3.2 I/min, without touching the swatch by hand. The pre-rub olfactive evaluation was performed on the swatches after 4 h. For this evaluation, the swatches were handled carefully in order to minimize the risk of breaking the microcapsules mechanically. The post-rub olfactive evaluation was performed after drying the swatches for 24 h at room temperature. This evaluation was performed by gently rubbing one part of each swatch.

**Table 2: Olfactive performance on terry toweling and hair swatch of freshly prepared and aged microcapsules**

| | Intensity on wet | Pre-rub intensity on dry | Post-rub intensity on dry |
|---|---|---|---|
| Laundry care conditioner | | | |
| Example 3 | 3.1 +/- 1 | 3 +/- 0.4 | 4.4 +/- 0.2 |
| Example 4.3 | 2.5 +/- 0.8 | 2.9 +/- 0.3 | 3.8 +/- 0.5 |
| Example 4.5 | 2.5 +/- 0.5 | 1.8 +/- 0.3 | 3.9 +/- 0.3 |
| Example 5.5 | 2.5 +/- 0.5 | 1.8 +/- 0.3 | 3.9 +/- 0.3 |
| Example 5.6 | 2.5 +/- 0.5 | 1.6 +/- 0.2 | 4.1 +/- 0.3 |
| Example 8 | 2.9 +/- 0.9 | 3.4 +/- 0.2 | 5 |

| Hair conditioner | | | |
|---|---|---|---|
| Example 3 | 2.8 +/- 0.7 | 4.5 +/- 0.5 | 5 |
| Example 4.3 | 3.9 +/- 0.5 | 2.8 +/- 0.5 | 4.4 +/- 0.5 |
| Example 8 | 3.5 +/- 0.3 | 4 +/- 0 | 5 |

The results show that microcapsules according to the present invention provide perfume performance that is comparable to conventional aminoplast-based microcapsules.

## Claims

1. An encapsulated composition comprising at least one core-shell microcapsule, wherein the at least one core-shell microcapsule comprises a core comprising at least one benefit agent and a shell surrounding the core, wherein the shell comprises a polymeric stabilizer that is formed by combination of a polymeric surfactant with at least one aminosilane, wherein the polymeric surfactant comprises a polysaccharide comprising carboxylic acid groups,
wherein the aminosilane is a bipodal aminosilane, wherein a bipodal aminosilane comprises at least one amino group and two residues, each of these residues bearing at least one alkoxysilane moiety.

2. An encapsulated composition according to claim 1, wherein the polysaccharide comprising carboxylic acid groups
a) comprises uronic acid units, in particular hexuronic acid units, optionally wherein the hexuronic acid units are selected from the group consisting of galacturonic acid units, glucuronic acid units, in particular 4-O-methyl-glucuronic acid units, guluronic acid units and mannuronic acid units; and/or
b) is branched.

3. An encapsulated composition according to one of claims 1 or 2, wherein the carboxylic acid groups are
a) partially present in the form of the corresponding methyl ester, optionally wherein the percentage of carboxylic acid groups that are present in the form of the corresponding methyl ester is from 3 % to 95 %, preferably from 4 % to 75 %, more preferably from 5 to 50 %; and/or
b) **at** least partially present in the form of the corresponding carboxylate salt, in particular the corresponding sodium, potassium, magnesium or calcium carboxylate salt.

4. An encapsulated composition according to one of claims 1 to 3, wherein the polysaccharide comprising carboxylic acid groups is at least partially acylated.

5. An encapsulated composition according to one of claims 1 to 4, wherein the polymeric surfactant is selected from pectin, gum arabic and an alginate.

6. An encapsulated composition according to one of claims 1 to 5, wherein the bipodal aminosilane is a secondary aminosilane, optionally wherein the secondary bipodal aminosilane is bis(3-(triethoxysilyl)propyl)amine.

7. An encapsulated composition according to one of claims 1 to 6, wherein the aminosilane to polymeric surfactant weight ratio is from 0.1 to 1.1, in particular from 0.2 to 0.9, even more particularly from 0.3 to 0.7.

8. An encapsulated composition according to one of claims 1 to 7, wherein the polymeric stabilizer is formed by combination of a polymeric surfactant with at least one bipodal aminosilane and further a polyfunctional isocyanate, optionally wherein the polyfunctional isocyanate is 2-ethylpropane-1,2,3-triyl tris((3-(isocyanatomethyl)phenyl)carbamate).

9. An encapsulated composition according to claims 5 and 6, wherein the polymeric stabilizer is formed by combination of pectin with bis(3-(triethoxysilyl)propyl)amine, and preferably additionally 2-ethylpropane-1,2,3-triyl tris((3-(isocyanatomethyl)phenyl)carbamate).

10. An encapsulated composition according to one of claims 1 to 9, comprising at least one core-shell microcapsule, wherein the at least one core-shell microcapsule comprises a core comprising at least one benefit agent and a shell surrounding the core, wherein the shell comprises a polymeric stabilizer that is formed by combination of a polymeric surfactant with at least one bipodal aminosilane, wherein the shell additionally comprises a polysaccharide, preferably a polysaccharide comprising beta (1 → 4) linked monosaccharide units, even more preferably a cellulose derivative, in particular selected form the group consisting of hydroxyethyl cellulose, hydroxypropylmethyl cellulose, cellulose acetate and carboxymethyl cellulose, preferably hydroxyethyl cellulose, optionally wherein the polysaccharide is deposited on the outer surface of the capsule shell formed by the polymeric stabilizer, optionally wherein the shell is further stabilized with a stabilizing agent, preferably comprising at least two carboxylic acid groups, even more preferably selected from the group consisting of citric acid, benzene-1,3,5-tricarboxylic acid, 2,5-furandicarboxylic acid, itaconic acid, poly(itaconic acid) and combinations thereof.

11. A method for preparing an encapsulated composition according to one of claims 1 to 10, the method comprising the steps of:
a) Providing a polymeric surfactant;
b) Providing an aqueous phase;
c) Dissolving or dispersing the polymeric surfactant in the aqueous phase;
d) Providing at least one aminosilane;
e) Providing an oil phase comprising at least one benefit agent;
f) Optionally: Dissolving the at least one aminosilane in the oil phase;
g) Emulsifying the oil phase and the aqueous phase in presence of both of the polymeric surfactant and the aminosilane to form an emulsion of oil droplets in the aqueous phase;
h) Causing the at least one aminosilane and the polymeric surfactant to form a shell at the oil-water interface of the emulsified oil droplets, thereby forming a slurry of microcapsules;
i) Optionally: Adding a polysaccharide, preferably a polysaccharide comprising beta (1 → 4) linked monosaccharide units, even more preferably a cellulose derivative, in particular selected form the group consisting of hydroxyethyl cellulose, hydroxypropylmethyl cellulose, cellulose acetate and carboxymethyl cellulose, preferably hydroxyethyl cellulose, to the microcapsule slurry formed in step h);
wherein the aminosilane is a bipodal aminosilane.

12. An encapsulated composition obtainable by the method according to claim 11.

13. Use of an encapsulated composition according to one of claims 1 to 10 or 12 to enhance the performance of a benefit agent in a consumer product.

14. A consumer product comprising an encapsulated composition according to one of claims 1 to 10 or 12, wherein the consumer product is preferably selected from the group consisting of fabric care detergents and conditioners, hair care conditioners, shampoos, heavy duty liquid detergents, hard surface cleaners, detergent powders, soaps, shower gels and skin care products.

15. A polymeric stabilizer formed by combination of a polymeric surfactant with at least one aminosilane, wherein the polymeric surfactant comprises a polysaccharide comprising carboxylic acid groups, wherein the aminosilane is a bipodal aminosilane, wherein a bipodal aminosilane comprises at least one amino group and two residues, each of these residues bearing at least one alkoxysilane moiety.

16. Use of a polymeric stabilizer according to claim 15 in the encapsulation of a benefit agent.

## Patentansprüche

1. Verkapselte Zusammensetzung, umfassend mindestens eine Kern-Schale-Mikrokapsel, wobei die mindestens eine Kern-Schale-Mikrokapsel einen Kern, der mindestens einen Effektstoff umfasst, und eine den Kern umgebende Schale umfasst, wobei die Schale einen durch Kombination eines polymeren Tensids mit mindestens einem Aminosilan gebildeten polymeren Stabilisator umfasst, wobei das polymere Tensid ein Polysaccharid mit Carbonsäuregruppen umfasst,
wobei es sich bei dem Aminosilan um ein bipodales Aminosilan handelt, wobei ein bipodales Aminosilan mindestens eine Aminogruppe und zwei Reste umfasst, wobei jeder dieser Reste mindestens eine Alkoxysilangruppierung trägt.

2. Verkapselte Zusammensetzung nach Anspruch 1, wobei das Polysaccharid mit Carbonsäuregruppen
a) Uronsäureeinheiten, insbesondere Hexuronsäureeinheiten, umfasst, gegebenenfalls wobei die Hexuronsäureeinheiten aus der Gruppe bestehend aus Galacturonsäureeinheiten, Glucuronsäureeinheiten, insbesondere 4-O-Methylglucuronsäureeinheiten, Guluronsäureeinheiten und Mannuronsäureeinheiten ausgewählt sind; und/oder
b) verzweigt ist.

3. Verkapselte Zusammensetzung nach einem der Ansprüche 1 oder 2, wobei die Carbonsäuregruppen
a) teilweise in Form des entsprechenden Methylesters vorliegen, gegebenenfalls wobei der Prozentanteil an Carbonsäuregruppen, die in Form des entsprechenden Methylesters vorliegen, 3 % bis 95 %, vorzugsweise 4 % bis 75 %, weiter bevorzugt 5 bis 50 %, beträgt; und/oder
b) mindestens teilweise in Form des entsprechenden Carboxylatsalzes, insbesondere des entsprechenden Natrium-, Kalium-, Magnesium- oder Calciumcarboxylatsalzes, vorliegen.

4. Verkapselte Zusammensetzung nach einem der Ansprüche 1 bis 3, wobei das Polysaccharid mit Carbonsäuregruppen mindestens teilweise acyliert ist.

5. Verkapselte Zusammensetzung nach einem der Ansprüche 1 bis 4, wobei das polymere Tensid aus Pektin, Gummi arabicum und einem Alginat ausgewählt ist.

6. Verkapselte Zusammensetzung nach einem der Ansprüche 1 bis 5, wobei es sich bei dem bipodalen Aminosilan um ein sekundäres Aminosilan handelt, gegebenenfalls wobei es sich bei dem sekundären bipodalen Aminosilan um Bis(3-(triethoxysilyl)propyl)amin handelt.

7. Verkapselte Zusammensetzung nach einem der Ansprüche 1 bis 6, wobei das Gewichtsverhältnis von Aminosilan zu polymerem Tensid 0,1 bis 1,1, insbesondere 0,2 bis 0,9, noch spezieller 0,3 bis 0,7, beträgt.

8. Verkapselte Zusammensetzung nach einem der Ansprüche 1 bis 7, wobei der polymere Stabilisator durch Kombination eines polymeren Tensids mit mindestens einem bipodalen Aminosilan und ferner einem polyfunktionellen Isocyanat gebildet wird, gegebenenfalls wobei es sich bei dem polyfunktionellen Isocyanat um 2-Ethylpropan-l,2,3-triyltris((3-(isocyanatomethyl)phenyl)carbamat) handelt.

9. Verkapselte Zusammensetzung nach den Ansprüchen 5 und 6, wobei der polymere Stabilisator durch Kombination von Pektin mit Bis(3-(triethoxysilyl)propyl)amin und vorzugsweise zusätzlich 2-Ethylpropan-l,2,3-triyltris((3-(isocyanatomethyl)phenyl)carbamat) gebildet wird.

10. Verkapselte Zusammensetzung nach einem der Ansprüche 1 bis 9, umfassend mindestens eine Kern-Schale-Mikrokapsel, wobei die mindestens eine Kern-Schale-Mikrokapsel einen Kern umfasst, der mindestens einen Effektstoff umfasst, und eine den Kern umgebende Schale umfasst, wobei die Schale einen durch Kombination eines polymeren Tensids mit mindestens einem bipodalen Aminosilan gebildeten polymeren Stabilisator umfasst, wobei die Schale zusätzlich ein Polysaccharid, vorzugsweise ein Polysaccharid mit beta-(1→4)-verknüpften Monosaccharideinheiten, noch weiter bevorzugt ein Cellulosederivat, das insbesondere aus der Gruppe bestehend aus Hydroxyethylcellulose, Hydroxypropylmethylcellulose, Celluloseacetat und Carboxymethylcellulose ausgewählt ist, vorzugsweise Hydroxyethylcellulose, umfasst, gegebenenfalls wobei das Polysaccharid auf der äußeren Oberfläche der durch den polymeren Stabilisator gebildeten Kapselschale abgeschieden ist, gegebenenfalls wobei die Schale ferner mit einem Stabilisierungsmittel, das vorzugsweise mindestens zwei Carbonsäuregruppen umfasst und noch weiter bevorzugt aus der Gruppe bestehend aus Citronensäure, Benzol-1,3,5-tricarbonsäure, 2,5-Furancarbonsäure, Itaconsäure, Poly(itaconsäure) und Kombinationen davon ausgewählt ist, stabilisiert ist.

11. Verfahren zur Herstellung einer verkapselten Zusammensetzung nach einem der Ansprüche 1 bis 10, wobei das Verfahren die folgenden Schritte umfasst:
a) Bereitstellen eines polymeren Tensids;
b) Bereitstellen einer wässrigen Phase;
c) Lösen oder Dispergieren des polymeren Tensids in der wässrigen Phase;
d) Bereitstellen mindestens eines Aminosilans;
e) Bereitstellen einer Ölphase, die mindestens einen Effektstoff umfasst;
f) gegebenenfalls: Lösen des mindestens einen Aminosilans in der Ölphase;
g) Emulgieren der Ölphase und der wässrigen Phase in Gegenwart sowohl des polymeren Tensids als auch des Aminosilans zur Bildung einer Emulsion von Öltröpfchen in der wässrigen Phase;
h) Bewirken, dass das mindestens eine Aminosilan und das polymere Tensid an der Öl-Wasser-Grenzfläche der emulgierten Öltröpfchen eine Schale bilden, wodurch eine Aufschlämmung von Mikrokapseln gebildet wird;
i) gegebenenfalls: Zugeben eines Polysaccharids, vorzugsweise eines Polysaccharids mit beta-(1 → 4)-verknüpften Monosaccharideinheiten, noch weiter bevorzugt eines Cellulosederivats, das insbesondere aus der Gruppe bestehend aus Hydroxyethylcellulose, Hydroxypropylmethylcellulose, Celluloseacetat und Carboxymethylcellulose ausgewählt ist, vorzugsweise Hydroxyethylcellulose, zu in Schritt h) gebildeten Mikrokapselaufschlämmung;
wobei es sich bei dem Aminosilan um ein bipodales Aminosilan handelt.

12. Verkapselte Zusammensetzung, die durch das Verfahren nach Anspruch 11 erhältlich ist.

13. Verwendung einer verkapselten Zusammensetzung nach einem der Ansprüche 1 bis 10 oder 12 zur Erhöhung der Leistungsfähigkeit eines Effektstoffs in einem Konsumprodukt.

14. Konsumprodukt, umfassend eine verkapselte Zusammensetzung nach einem der Ansprüche 1 bis 10 oder 12, wobei das Konsumprodukt vorzugsweise aus der Gruppe bestehend aus Textilwaschmitteln und -weischpülern, Haarpflegespülungen, Shampoos, Flüssigvollwaschmitteln, Reinigungsmitteln für harte Oberflächen, Waschmittelpulvern, Seifen, Duschgelen und Hautpflegeprodukten ausgewählt ist.

15. Polymerer Stabilisator, gebildet durch Kombination eines polymeren Tensids mit mindestens einem Aminosilan, wobei das polymere Tensid Polysaccharid mit Carbonsäuregruppen umfasst, wobei es sich bei dem Aminosilan um ein bipodales Aminosilan handelt, wobei ein bipodales Aminosilan mindestens eine Aminogruppe und zwei Reste umfasst, wobei jeder dieser Reste mindestens eine Alkoxysilangruppierung trägt.

16. Verwendung eines polymeren Stabilisators nach Anspruch 15 bei der Verkapselung eines Effektstoffs.

## Revendications

1. Composition encapsulée comprenant au moins une microcapsule noyau-enveloppe, dans laquelle l'au moins une microcapsule noyau-enveloppe comprend un noyau comprenant au moins un agent bénéfique et une enveloppe entourant le noyau, dans laquelle l'enveloppe comprend un stabilisant polymérique qui est formé par combinaison d'un tensioactif polymérique avec au moins un aminosilane, dans laquelle le tensioactif polymérique comprend un polysaccharide comprenant des groupes acide carboxylique,
dans laquelle l'aminosilane est un aminosilane bipodal, dans laquelle un aminosilane bipodal comprend au moins un groupe amino et deux radicaux, chacun de ces radicaux portant au moins un groupement alcoxysilane.

2. Composition encapsulée selon la revendication 1, dans laquelle le polysaccharide comprenant des groupes acide carboxylique
a) comprend des unités acide uronique, notamment des unités acide hexuronique, éventuellement les unités acide hexuronique étant choisies dans le groupe constitué par les unités acide galacturonique, les unités acide glucuronique, en particulier les unités acide 4-O-méthyl-glucuronique, les unités acide guluronique et les unités acide mannuronique ; et/ou
b) est ramifié.

3. Composition encapsulée selon l'une des revendications 1 ou 2, dans laquelle les groupes acide carboxylique sont
a) partiellement présents sous la forme de l'ester méthylique correspondant, éventuellement, le pourcentage de groupes acide carboxylique qui sont présents sous la forme de l'ester méthylique correspondant étant de 3 % à 95 %, préférablement de 4 % à 75 %, plus préférablement de 5 à 50 % ; et/ou
b) au moins partiellement présents sous la forme de sel de carboxylate correspondant, en particulier le sel de carboxylate de sodium, de potassium, de magnésium ou de calcium correspondant.

4. Composition encapsulée selon l'une des revendications 1 à 3, dans laquelle le polysaccharide comprenant des groupes acide carboxylique est au moins partiellement acylé.

5. Composition encapsulée selon l'une des revendications 1 à 4, dans laquelle le tensioactif polymérique est choisi parmi la pectine, la gomme arabique et un alginate.

6. Composition encapsulée selon l'une des revendications 1 à 5, dans laquelle l'aminosilane bipodal est un aminosilane secondaire, éventuellement dans laquelle l'aminosilane bipodal secondaire est la bis(3-(triéthoxysilyl)propyl)amine.

7. Composition encapsulée selon l'une des revendications 1 à 6, dans laquelle le rapport pondéral aminosilane sur tensioactif polymérique est de 0,1 à 1,1, en particulier de 0,2 à 0,9, plus particulièrement encore de 0,3 à 0,7.

8. Composition encapsulée selon l'une des revendications 1 à 7, dans laquelle le stabilisant polymérique est formé par combinaison d'un tensioactif polymérique avec au moins un aminosilane bipodal et en outre un isocyanate polyfonctionnel, éventuellement dans laquelle l'isocyanate polyfonctionnel est le tris((3-(isocyanatométhyl)phényl)carbamate) de 2-éthylpropane-1,2,3-triyle.

9. Composition encapsulée selon les revendications 5 et 6, dans laquelle le stabilisant polymérique est formé par combinaison de pectine avec la bis(3-(triéthoxysilyl)propyl)amine, et de préférence en outre le tris((3-(isocyanatométhyl)phényl)carbamate) de 2-éthylpropane-1,2,3-triyle.

10. Composition encapsulée selon l'une des revendications 1 à 9, comprenant au moins une microcapsule noyau-enveloppe, dans laquelle l'au moins une microcapsule noyau-enveloppe comprend un noyau comprenant au moins un agent bénéfique et une enveloppe entourant le noyau, dans laquelle l'enveloppe comprend un stabilisant polymérique qui est formé par combinaison d'un tensioactif polymérique avec au moins un aminosilane bipodal, dans laquelle l'enveloppe comprend en outre un polysaccharide, de préférence un polysaccharide comprenant des unités monosaccharide à liaison bêta (1 → 4), encore plus préférablement un dérivé cellulosique, en particulier choisi dans le groupe constitué par l'hydroxyéthylcellulose, l'hydroxypropylméthylcellulose, l'acétate de cellulose et la carboxyméthylcellulose, de préférence l'hydroxyéthylcellulose, éventuellement dans laquelle le polysaccharide est déposé sur la surface externe de l'enveloppe de capsule formée par le stabilisant polymérique, éventuellement dans laquelle l'enveloppe est en outre stabilisée par un agent stabilisant, de préférence comprenant au moins deux groupes acide carboxylique, encore plus préférablement choisis dans le groupe constitué par l'acide citrique, l'acide benzène-1,3,5-tricarboxylique, l'acide 2,5-furanedicarboxylique, l'acide itaconique, un poly(acide itaconique) et leurs combinaisons.

11. Procédé de préparation d'une composition encapsulée selon l'une des revendications 1 à 10, le procédé comprenant les étapes de :
a) fourniture d'un tensioactif polymérique ;
b) fourniture d'une phase aqueuse ;
c) dissolution ou dispersion du tensioactif polymérique dans la phase aqueuse ;
d) fourniture d'au moins un aminosilane ;
e) fourniture d'une phase huileuse comprenant au moins un agent bénéfique ;
f) éventuellement : dissolution de l'au moins un aminosilane dans la phase huileuse ;
g) émulsification de la phase huileuse et de la phase aqueuse en présence d'à la fois le tensioactif polymérique et l'aminosilane pour former une émulsion de gouttelettes d'huile dans la phase aqueuse ;
h) le fait de provoquer la formation d'une enveloppe, à partir de l'au moins un aminosilane et du tensioactif polymérique, au niveau de l'interface huile-eau des gouttelettes d'huile émulsifiées, formant ainsi une suspension de microcapsules ;
i) éventuellement : ajout d'un polysaccharide, de préférence un polysaccharide comprenant des unités monosaccharide à liaison bêta (1 → 4), encore plus préférablement un dérivé cellulosique, en particulier choisi dans le groupe constitué par l'hydroxyéthylcellulose, l'hydroxypropylméthylcellulose, l'acétate de cellulose et la carboxyméthylcellulose, de préférence l'hydroxyéthylcellulose, dans la suspension de microcapsules formée à l'étape h) ;
dans laquelle l'aminosilane est un aminosilane bipodal.

12. Composition encapsulée pouvant être obtenue par le procédé selon la revendication 11.

13. Utilisation d'une composition encapsulée selon l'une des revendications 1 à 10 ou 12 pour améliorer la performance d'un agent bénéfique dans un produit de consommation.

14. Produit de consommation comprenant une composition encapsulée selon l'une des revendications 1 à 10 ou 12, dans lequel le produit de consommation est de préférence choisi dans le groupe constitué par les détergents et les conditionneurs d'entretien des tissus, les après-shampooings, les shampooings, les détergents puissants, les nettoyants pour surfaces dures, les poudres détergentes, les savons, les gels douches et les produits de soins de la peau.

15. Stabilisant polymérique formé par combinaison d'un tensioactif polymérique avec au moins un aminosilane, dans lequel le tensioactif polymérique comprend un polysaccharide comprenant des groupes acide carboxylique, dans lequel l'aminosilane est un aminosilane bipodal, dans lequel un aminosilane bipodal comprend au moins un groupe amino et deux radicaux, chacun de ces radicaux portant au moins un groupement alcoxysilane.

16. Utilisation d'un stabilisant polymérique selon la revendication 15 dans l'encapsulation d'un agent bénéfique.
